# EUROPÄISCHE PATENTSCHRIFT

(11) **EP 1 477 152 B1**
(45) Veröffentlichungstag und Bekanntmachung des Hinweises auf die Patenterteilung: **23.06.2010**
(21) Anmeldenummer: 04009480.7
(22) Anmeldetag: 22.04.2004
(51) Int. Cl.: A61Q 19/00, A61Q 19/02, A61Q 19/04, A61Q 19/08, A61K 8/11, A61K 8/34, A61K 8/86

(54) **Trägersystem für kosmetische oder pharmazeutische Wirkstoffe**
Excipient system for cosmetic or pharmaceutical active substances
Système d'excipient pour agents actifs cosmétiques ou pharmaceutiques

(30) Priorität: 12.05.2003 DE 10321145
(43) Veröffentlichungstag der Anmeldung: 17.11.2004
(73) Patentinhaber: Beiersdorf AG, 20253 Hamburg (DE)
(72) Erfinder: Raschke, Thomas, 25421 Pinneberg (DE); Teuber, Frank, 22846 Nordestedt (DE); Bellwinkel, Klaus, 32427 Minden (DE); Otto, Sebastian, 20535 Hamburg (DE)

(56) Entgegenhaltungen:
- EP-A- 1 243 323
- EP-A- 1 243 326
- EP-A- 1 428 571
- WO-A-02/50230
- US-A- 4 152 272
- US-A1- 2003 053 974
- US-B1- 6 362 146

## Beschreibung

Die Erfindung umfasst ein Trägersystem für kosmetische oder pharmazeutische Wirkstoffe bestehend aus Wachspartikeln und Solubilisatoren. Das erfindungsgemäße Trägersystem umfasst 0,01 - 15 Gew.% Solubilisatoren, die gewählt werden aus der Gruppe der (a) Polyole, (b) Polyethylenglycole, und/oder (c) Wollwachsalkohole.

Im Rahmen der vorliegenden Offenbarung wird als Oberbegriff für Fette, Öle, Wachse und dergleichen der Ausdruck "Lipide" verwendet, wie dem Fachmann durchaus geläufig ist. Auch werden die Begriffe "Ölphase" und "Lipidphase" synonym angewandt.

Zahlreiche kosmetische und/oder dermatologische Wirkstoffe sind gegenüber bestimmten Einflüssen wie Feuchtigkeit, niedrigen oder hohen pH-Werten sowie Sauerstoff nicht stabil. Es hat daher nicht an Versuchen gefehlt, derartige Wirkstoffe den Umwelteinflüssen so zu entziehen, so dass dennoch bei der Anwendung eine Freisetzung der Wirkstoffe stattfindet. Ein Weg zur Erreichung dieses Ziels ist die Verkapselung von Wirkstoffen. Das Verkapselungsmaterial bildet damit ein Trägersystem für die Wirkstoffe, um sie vor Umwelteinflüssen zu schützen und in geeignete Zubereitungen einzuarbeiten.

Zur Herstellung verkapselter kosmetischer Wirkstoffe gibt es mehrere Ansätze. Bekannt ist beispielsweise die liposomale Verkapselung von Arzneistoffen, die zu einer retardierten Wirkstofffreisetzung führen soll. Im wesentlichen handelt es sich dabei um von Phospholipiden umschlossene wirkstoffhaltige spherische Vesikel, die sogenannten Liposome. Die Langzeitstabilität derartiger Gebilde ist jedoch gering.
Nanopartikel sind feste Partikel mit Partikelgrößen von 15 bis 300 nm. Gelegentlich werden auch größere Teilchen mit Durchmessern bis zu 1000 nm zu den Nanopartikeln gerechnet. Derartige Partikel bestehen in der Regel aus Polymeren und weisen Kavitäten auf oder bilden eine Hülle, so dass sich in ihrem Inneren Gastmoleküle aufhalten können, die umschlossen oder adsorbiert werden. Diese Gastmoleküle können auch Wirkstoffe sein, die bei Anwendung der Nanopartikel langsam freigesetzt werden.
Ähnlich verhalten sich feste Lipidnanopartikel, die in einer Matrix aus festen Lipiden verteilte Wirkstoffe enthalten. Die Größe der Partikel ist mit denen der Nanopartikel vergleichbar.

Zur Verkapselung von pharmazeutischen oder kosmetischen Wirkstoffen zur Kontrolle der Wirkstofffreisetzung oder der stabilen Einarbeitung in Zubereitungen sind zahlreiche Methoden bekannt.
Die europäische Patentanmeldung EP 1064912 offenbart Wirkstoff enthaltende Mikrokapseln mit einem Durchmesser von 0,1 bis 5 mm, die man erhält, in dem man aus Gelbildnern, Chitosan und Wirkstoff eine Matrix herstellt, und diese in wäßrige Lösungen anionischer Polymere eintropft. Dabei bildet sich aus Chitosan und anionischem Polymer eine Membran, die die Wirkstofflösung umhüllt. Bekannt sind auch, beispielsweise unter der Bezeichnung Retinol-Primasphere bzw. Vitamin E-Primasphere, chitosanverkapseltes Retinol bzw. D-alpha-Tocopherol mit Partikeldurchmessern von 5 bis 10 µm. Die europäische Patentanmeldung EP 1129771 beschreibt eine Variante dieses Verfahrens, bei dem zunächst eine wirkstoffhaltige O/W-Emulsion hergestellt wird, anschließend Lösungen anionischer Polymere und abschließend Chitosan zugegeben werden, um die Mikrokapseln zu erhalten.
In Colloid Polym. Sci. 275 46-53 (1997) werden ähnliche Nanokapseln aus anionische Polymere enthaltenden Kolloiden, die mit Chitosan beschichtet werden, beschrieben. Diese weisen Partikeldurchmesser von 200 bis 500 nm auf.
In J. Controlled Release 39 (1996) 17-25 werden den pharmazeutischen Wirkstoff Prednisolon enthaltende Chitosan-Mikrokapseln beschrieben, die durch Zugabe einer Salzlösung aus einer wäßrigen Chitosanlösung erhalten wurden.
Die deutsche Offenlegungsschrift DE 19810965 beschreibt Nanopartikel enthaltend einen Wirkstoff, einen Komplex aus Polyanion und Polykation sowie einem Vernetzungsmittel. Diese weisen Partikeldurchmesser von 50 bis 250 nm auf.
Allgemeines zu Verkapselungstechniken mit Chitosan findet sich in J Microencapsulation 14 Seiten 689-711 (1997).

Zwar sind in Lipidpartikeln verkapselte, lipophile Wirkstoffe an sich dem Fachmann bekannt. So beschreiben die EP 167825, DE 100 59 668, DE 199 45 203, EP 0934743, WO 94/20072, WO 00/10522 sowie die WO 00/67728 wirkstoffbeladene Lipidpartikel.

EP 1243323 offenbart Nanokapseln die aus Lecithinen, Phospholipiden, Wachskörpern und Wirkstoffen bestehen. Den Kapseln können zur Herstellung Co-Lösemittel, wie kurzkettige Alkohole, zugesetzt werden.

In der EP 1428571 werden Mikrokapseln beschrieben, die neben Wirkstoffen Polyethylenglykolwachse, Ölkörper und Emulgatoren enthalten.

Doch konnten diese Schriften nicht das Problem der Verkapselung von in Wachspartikeln schwerlöslichen, insbesondere hydrophilen Wirkstoffen lösen.

Denn vor allem viele wichtige kosmetische Wirkstoffen sind hydrophil, wasserlöslich oder sauer, wie beispielsweise Isoflavonoide, Flavonoide, wässrige Pflanzenextrakte, organische Säuren, Amine. Keine der angegebenen Schriften offenbart einen Weg zur Verkapselung hydrophiler kosmetischer Wirkstoffe in Lipiden. Dies ist aber in hohem Maße wünschenswert, da viele dieser Stoffe nicht stabil in die üblichen kosmetischen und/oder dermatologischen Zubereitungen einzuarbeiten sind oder aber die Zubereitungen destabilisieren oder die Zersetzung anderer Ingredienzien bewirken. Dies ist insbesondere der Fall, wenn Pigmente in den Zubereitungen enthalten sind.

Die bekannten Wirkstoffträgersysteme eignen sich aufgrund des Trägermaterials "Wachs" eigentlich nur für lipophile Wirkstoffe. Zudem ist die Gesamtmenge an Wirkstoff, die stabil in die Wachsmatrix eingearbeitet werden kann, stark limitiert.

Aufgabe der vorliegenden Erfindung ist es, ein Trägersystem bereit zu stellen, dass es ermöglicht, Wirkstoffe in Wachspartikel einzubauen, ohne dass sie die aus dem Stand der Technik bekannten Nachteile aufweisen. Insbesondere gilt es, Trägersysteme bereit zu stellen, die es ermöglichen hydrophile Wirkstoffe stabil in Wachspartikel einzubauen.

Weiterhin ist es die Aufgabe der vorliegenden Erfindung eine Steigerung der Wirkstoffkonzentration im Trägersystem, der Wachspartikel, zu erzielen, um die Leistungsfähigkeit der wirkstoffbeladenen Partikel zu erhöhen.
Aufgabe der Erfindung ist es auch kosmetische Zubereitungen bereit zu stellen, die eigentlich in Wachspartikeln schwerlösliche kosmetische, dermatologische oder pharmazeutische Wirkstoffe enthalten.

Gelöst wird dieses Bündel an Aufgaben durch ein Trägersystem bzw. einer kosmetischen Zubereitung entsprechend den Hauptansprüchen. In den Unteransprüchen sind bevorzugte Ausführungsformen des Systems bzw. der Zubereitungen offenbart. Die Erfindung umfasst darüber hinaus auch die Verwendung des Trägersystems.
Es war überraschend und für den Fachmann außerordentlich erstaunlich, dass ein Trägersystem für kosmetische oder pharmazeutische Wirkstoffe, umfassend Wachsmikro- oder -nanopartikel aus einem oder mehreren Wachsen oder Wachs/Ölgemischen enthaltend 0,01 bis 15 Gew.%, bezogen auf die Wachsphase, einen oder mehrere Solubilisatoren gewählt aus der Gruppe der (a) Polyole, (b) Polyethylenglycole und/oder (c) Wollwachsalkohole und einen oder mehrere kosmetische und/oder pharmazeutische Wirkstoffe, das Bündel an Aufgaben löst.

Überraschenderweise hat sich insbesondere gezeigt, dass mit dem erfindungsgemäßen Trägerssystem hydrophile Wirkstoffe mit einem log *P*_{*Octanol*/*Wasser*} kleiner 3 stabil in kosmetischen Zubereitungen eingearbeitet und diese lager- und anwendungsstabil zur Verfügung gestellt werden können.

Insbesondere die Verkapselung von Wirkstoffen mit hydrolyseempfindlichen funktionellen Gruppen, wie beispielsweise die Acetyl-, Glucosyl- , Galactosyl-, EthoxyGruppe (beispielsweise als Bestandteil von Estern), Benzyl- und Benzoyl-Gruppen sowie Ester mit Kohlenwasserstoffrest oder einer Aminosäure als Restfunktion, ist mit dem erfindungsgemäßen System vorteilhaft.
Bevorzugte Wirkstoffe bzw. Wirkstoffgruppen im Sinne der Erfindung sind Acetylcarnitin, Acetylsalicylsäure, Liponamid, Pflanzenextrakte mit phenolischen oder polyphenolischen Wirkkomponenten (z.B. Grüner Tee, Isoflavonoide), Harnsäure und seine Derivate, Flavonoide insbesondere Quercetin und seine Derivate, Dihydroxyaceton und deren Derivate.
Es war äußerst erstaunlich, und vielfältig durchgeführte Untersuchung belegen dies, dass ein Zusatz von Polyolen, Polyethylenglycolen und/oder Wollwachsalkoholen in die Wachsmatrix die Einarbeitung von hydrophilen oder ansonsten in Wachsmatrices schwerlöslichen Wirkstoffen verbessert bzw. überhaupt erst ermöglicht.

Der Zusatz von Polyolen und anderen Solubilisatoren in kosmetischen Zubereitungen ist bekannt und findet üblicherweise in der wässrigen Phase statt und dient in erster Linie der Hautpflege. Erfindungsgemäß sind die Polyole jedoch in der Wachsmatrix enthalten und nicht in der wässrigen Phase und sind damit für die hautpflegende Wirkungsweise entzogen.

Die Einarbeitung der erfindungsgemäßen Solubilisatoren, Polyole, Polyethylenglycole und/oder Wollwachsalkohole, in Wachsmatrices ist nicht naheliegend, da Wachs bekanntermaßen nur lipophile Stoffe bereitwillig aufzunehmen vermag. Durch die Kombination von Wachsen, insbesondere ausgewählten Wachsen mit bestimmten, polaren Strukturkomponenten, wie beispielsweise Ester, mit den Solubilisatoren, den Polyole, Polyethylenglycol und/oder Wollwachs, wird die Wachsmatrixstruktur tendenziell etwas polarer, so dass die Möglichkeit eröffnet wird, hydrophile Wirkstoffe in die Wachsmatrix einzubauen und gleichzeitig ein Auskristallisieren des Wirkstoffes in der Wachsmatrix vermindert wird.

Ein weitere Vorteil ist, dass die Solubilisatoren, insbesondere die Polyole, in relativ geringen Menge eingesetzt werden können und dennoch den gewünschten Effekt bewirken. Die Solubilisatoren werden in einem Anteil von 0,01 bis 15 Gew.%, bezogen auf die Gesamtmasse der Wachsmatrix, eingesetzt, besonders bevorzugt im Bereich 0,1-10 Gew.%.

Als besonders bevorzugte Vertreter aus der Gruppe (a) der Polyole können folgende Substanzen gewählt werden: Butylenglycol, Dipropylenglycol, Propylenglycol und/oder Polyolprepolymer (PP-2).

Neben den Polyolen können erstaunlicherweise auch Wollwachsalkohole, wie das bekannte Eucerit, als Zusatz in den Wachspartikeln eingesetzt werden, um die Aufnahmekapazität von hydrophilen Wirkstoffen zu verbessern.

Des weiteren haben sich Polyethylenglycole als vorteilhafte Solubilisatoren im Sinne der Erfindung gezeigt. Vorteilhaft lassen sich Polyethylenglycole mit einem mittleren Ethylenglycolanteil von 8-500 Einheiten einsetzen.

Vertreter der drei erfindungsgemäßen Solubilisatorengruppen a), b) und c) können allein oder gemischt eingesetzt werden.

Das erfindungsgemäße System setzt sich bevorzugt aus Wachse zusammen, die aus der Gruppe der
• natürlichen Wachse, besonders bevorzugt Carnaubawachs, Candelillawachs, Shellackwachs, Beerenwachs (Rhus Verniciflura), hydrierte Pflanzenöle, wie hydriertes Palm- oder Rapsöl
• Mono-, Di - und Triglyceride aus höheren gesättigten Fettsäuren mit 10 - 30 Kohlenstoffatomen, besonders bevorzugt Glyceryltripalmitat (Dynasan 116) und/oder Glycerylstearat, Kahlwachs 6447 (Gemisch aus Fettsäureestern und Kohlenwasserstoffpolymer)
• höherer gesättigter Fettalkohole, besonders bevorzugt solche mit 14 - 30 Kohlenstoffatomen, ganz besonders bevorzugt Stearylalkohol und/oder Behenylalkohol
• synthetischer Ester, bevorzugt C16-36 Alkylhydroxystearoylstearat, Stearylstearat, Cetearylbehenat, C20-40 Alkylstearat, besonders bevorzugt Cetylpalmitat
• Polymerwachse, bevorzugt Polyethylen, Polypropylen, Polyvinylether, besonders bevorzugt Polyvinylstearylether ,
• Copolymere, besonders bevorzugt solche aus Ethylen- und Vinylacetat,
• Kohlenwasserstoffe/ Paraffinwachse, besonders bevorzugt Cera Microcristallina, Paraffinwachs,
gewählt werden.

Erfindungsgemäß besonders bevorzugte Wachse zur Herstellung der erfindungsgemäßen Wachsmatrix sind Cetylpalmitat, Candellilawachs, Carnaubawachs, Paraffinwachs, Tripalmitin (Dynasan 116), Kahlwachs 6447 und C15-C40 Alkyl Stearylstearate, die im Handel unter den Handelsnamen Kesterwachs K48P, K70P, K80P, erhältlich sind.

Erfindungsgemäß vorteilhaft sind auch natürliche Wachse tierischen und pflanzlichen Ursprungs, wie beispielsweise Bienenwachs und andere Insektenwachse sowie Beerenwachs (Rhus Verniciflura).

Die Wachsmatrix kann partikulär, als Pulver oder als Suspension in einem polaren Lösungsmittel, z.B. Wasser, vorliegen. Partikulär weisen die Wachspartikel bevorzugt eine Partikelgröße von 0,01 bis 250 µm auf. Die mittlere Partikelgröße beträgt vorzugsweise 0,3 bis 30 µm.

Wachspartikel im Sinne der vorliegenden Erfindung weisen vorteilhaft einen Schmelzpunkt von 20 bis 90 °C, bevorzugt einen Schmelzpunkt von 30 bis 80 °C und besonders bevorzugt einen Schmelzpunkt von 40 bis 70°C auf.

Die Wirkstoffe, die im Trägersystem aufgenommen werden, können aus den kosmetisch, dermatologisch und pharmazeutischen Wirkstoffen gewählt werden, wobei bevorzugt aus den folgenden Wirkstoffgruppen ausgewählt werden kann:
Kosmetik-Wirkstoffe: Carnitin und Derivate, Creatin und Derivate, Liponamid, Salicylsäure und Derivate wie z.B. Acetylsalicylsäure, Isoflavonoide, wie beispielsweise Genistein und Daidzein, Polyphenole des Grünem Tees (Camellia sinensis, Dragoco), insbesondere die Wirkkomponente Epigallocatechingallat, sowie Rutin und seine Derivate, Ester und/oder Amide von Aminosäuren, Peptide und deren Derivate bestehend aus 2-10 Aminosäuren, Ascorbinsäure und/oder Derivate, Vitamine der B-Gruppe, insbesondere Niacinamid und Pyridoxin, sowie Folsäure.
Pharmawirkstoffe: Analgetika/Antirheumatika (z.B. Ibuprofen, Diclofenac), Peptidhormone, Vasodilatoren, Antiallergica (z.B. Terfenadin), Antibiotika/Chemotherapeutika (z.B. aus den Gruppen der Makrolide, Aminoglycoside, Cefalosporine, Penicilline, Polypeptide, Tetracycline), Virustatika Antimykotika, Opthalmika, Psychopharmaka, Immunstoffe und Cytostatika.

Das erfindungsgemäße Trägersystem führt zu folgenden Vorteilen und Verbesserungen:
- Verbesserung der Lagerstabilität von oxidations- und hydrolyseempfindlichen Wirkstoffen
- Reduktion des Wirkstoffabbaus unter Herstellbedingungen des Trägersystems, da die Einarbeitungstemperatur für sensible Wirkstoffe deutlich reduziert werden kann.
- Reduktion der Freisetzungsgeschwindigkeit des Wirkstoffes aus der Rezeptur
- Verbesserung der lokalen Verträglichkeit des verkapselten Wirkstoffes sowie Verlängerung der Wirkdauer
- in kosmetischen Zubereitungen - Verbesserung der Verteilbarkeit des Wirkstoffes auf der Oberfläche des Anwendungsareals (z.B. Haut) und somit Verbesserung der Wirkung
- Verstärkung der Wirkung durch Steigerung der Wirkstoffpenetration am Wirkort infolge Okklusion durch das Vehikel (Wachspartikel)
- Vermeidung unerwünschter Wechselwirkungen zwischen Wirkstoff und anderen Rezepturbestandteilen (Konservierungsmitteln, Verdickern, Füllstoffen und Additiven wie Metalloxiden) der kosmetischen Zubereitung

Bei der Suspendierung der Wachspartikel in eine wässrige Phase kann unter Umständen der Einsatz von einem oder mehreren oberflächenaktiven Verbindungen erfindungsgemäß vorteilhaft oder gar notwendig sein.

Bevorzugte oberflächenaktive Verbindungen sind O/W-Emulgatoren.
O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel

   R-O-(-CH₂-CH₂-O-)ₙ-R',
- der Fettsäureethoxylate der allgemeinen Formel

   R-COO-(-CH₂-CH₂-O-)ₙ-H,
- der veretherten Fettsäureethoxylate der allgemeinen Formel

   R-COO-(-CH₂-CH₂-O-)ₙ-R',
- der veresterten Fettsäureethoxylate der allgemeinen Formel

   R-COO-(-CH₂-CH₂-O-)ₙ-C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel

   R-O-(-CH₂-CH₂-O-)ₙ-CH₂-COOH

   nd n eine Zahl von 5 bis 30 darstellen,
- der Polyoxyethylensorbitolfettsäureester,
- der Alkylethersulfate der allgemeinen Formel

   R-O-(-CH₂-CH₂-O-)ₙ-SO₃-H
- der Fettalkoholpropoxylate der allgemeinen Formel

   R-O-(-CH₂-CH(CH₃)-O-)ₙ-H,
- der Polypropylenglycolether der allgemeinen Formel

   R-O-(-CH₂-CH(CH₃)-O-)ₙ-R',
- der propoxylierten Wollwachsalkohole,
- der veretherten Fettsäurepropoxylate

   R-COO-(-CH₂-CH(CH₃)-O-)ₙ-R',
- der veresterten Fettsäurepropoxylate der allgemeinen Formel

   R-COO-(-CH₂-CH(CH₃)-O-)ₙ-C(O)-R',
- der Fettsäurepropoxylate der allgemeinen Formel

   R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

   R-O-(-CH₂-CH(CH₃)O-)ₙ-CH₂-COOH
- der Alkylethersulfate bzw. die diesen Sulfaten zugrundeliegenden Säuren der allgemeinen Formel

   R-O-(-CH₂-CH(CH₃)-O-)ₙ₋SO₃-H
- der Fettalkoholethoxylate/propoxylate der allgemeinen Formel

   R-O-Xₙ-Yₘ-H,
- der Polypropylenglycolether der allgemeinen Formel

   R-O-Xₙ-Yₘ-R'.
- der veretherten Fettsäurepropoxylate der allgemeinen Formel

   R-COO-Xₙ-Yₘ-R',
- der Fettsäureethoxylate/propoxylate der allgemeinen Formel

   R-COO-Xₙ-Yₘ-H,.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11-18. ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:

Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol-(18)stearylether (Steareth-18), Polyethylenglycol(19)stearylether (Steareth-19), Polyethylenglycol(20)stearylether (Steareth-20),

Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol-(18)isostearylether (Isosteareth-18), Polyethylenglycol(19)isostearylether (Isosteareth-19), Polyethylenglycol(20)isostearylether (Isosteareth-20),

Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(1 8)cetylether (Ceteth-18), Polyethylenglycol(19)cetylether (Ceteth-19), Polyethylenglycol(20)cetylether (Ceteth-20),

Polyethylenglycol(1 3)isocetylether (Isoceteth-13), Polyethylenglycol(14)isocetylether (Isoceteth-14), Polyethylenglycol(15)isocetylether (Isoceteth-15), Polyethylenglycol(16)-isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),

Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15)

Polyethylenglycol(12)laurylether (Laureth-12), Polyethylenglycol(12)isolaurylether (Isolaureth-12).

Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol(19)-cetylstearylether (Ceteareth-19), Polyethylenglycol(20)cetylstearylether (Ceteareth-20),

Es ist ferner von Vorteil, die Fettsäureethowlate aus folgender Gruppe zu wählen.

Polyethylenglycol(20)stearat, Polyethylenglycol(21) stearat, Polyethylenglycol(22)stearat, Polyethylenglycol(23)stearat, Polyethylenglycol(24)stearat, Polyethylenglycol(25)stearat, Polyethylenglycol(12)isostearat, Polyethylenglycol(13)isostearat, Polyethylenglycol(14)-isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21 )isostearat, Polyethylenglycol-(22)isostearat, Polyethylenglycol(23)isostearat, Polyethylenglycol(24)isostearat, Polyethylenglycol(25)isostearat,

Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol-(22)glyceryllaurat, Polyethylenglycol(23)glyceryllaurat, Polyethylenglycol(6)glycerylcaprat/caprinat. Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Besonders bevorzugte O/W-Emulgatoren sind Triceteareth-4-Phosphat, Polyglyceryl-3-Methylglucosedistearat, Polyethylenglycol-40-Stearat.

Das erfindungsgemäße Trägersystem wird bevorzugt in kosmetischen und/oder dermatologischen Zubereitungen eingesetzt, so dass eine bevorzugte Ausführungsform der Erfindung kosmetische und/oder dermatologische wirkstoffhaltige Zubereitungen sind, die erfindungsgemäßes Trägersystem enthalten.
Diese Zubereitungen werden bevorzugt verwendet zur Pflege der Haut, zur Vorbeugung der Hautalterung, zur Verringerung von Falten, zum Schutz der Haut vor exogenen Noxen, zur Barrieresteigerung der Haut, zur Hautaufhellung, zur Hautbräunung, zur Vermeidung oder Reduktion von Hautirritationen oder entzündlichen Prozessen bzw. Hautrötungen sowie zur Beseitigung bzw. Reduzierung von Körpergerüchen sowie zur gezielten Behandlung von Haaren und der Haarwurzeln.

Die erfindungsgemäßen kosmetischen und/oder dermatologischen Zubereitungen können in verschiedenen Formen vorliegen können. So können sie z. B. eine wässrige Dispersion, eine Emulsion vom Typ Öl-in-Wasser (O/W), oder eine multiple Emulsionen, beispielsweise vom Typ Wasser-in-Öl-in-Wasser (W/O/W), eine Hydrodispersion, ein Gel, eine Pickering-Emulsion, einen festen Stift oder auch ein Aerosol darstellen.
Erfindungsgemäß vorteilhafte Darreichungsformen der erfindungsgemäßen Zubereitungen sind Cremes, Salben, Hydrodispersionen, Lotionen, Tinkturen, Pumpsprays, Aerosolsprays, wässrige Lösungen und dergleichen.

Es ist dem Fachmanne natürlich bekannt, dass anspruchsvolle kosmetische Zusammensetzungen zumeist nicht ohne die üblichen Hilfs- und Zusatzstoffe denkbar sind. Die erfindungsgemäßen kosmetischen Zubereitungen können daher kosmetische Hilfsstoffe enthalten, wie sie üblicherweise in solchen Zubereitungen verwendet werden, z.B. Konservierungsmittel, Bakterizide, desodorierend wirkende Substanzen, Antitranspirantien, Insektenrepellentien, weitere Vitamine, Mittel zum Verhindern des Schäumens, Farbstoffe, Pigmente mit färbender Wirkung, Verdickungsmittel, weichmachende Substanzen, anfeuchtende und/oder feuchthaltende Substanzen, Antioxidantien, UV-Filtersubstanzen, Filmbildner, Tenside, Fette, Öle, Wachse oder andere übliche Bestandteile einer kosmetischen Formulierung wie Alkohole, Polyole, wobei diese in der Zubereitung vorhanden sind und nicht mit denjenigen im erfindungsgemäße Trägersystem verwechselt werden dürfen, Polymere, Schaumstabilisatoren, Elektrolyte, organische Lösungsmittel oder Silikonderivate.

Die wässrige Phase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Celluloseether wie z.B. Hydroxypropylmethylcellulose, besonders vorteilhaft aus der Gruppe der Polyacrylate, bevorzugt ein Polyacrylat aus der Gruppe der sogenannten Carbopole [von der Fa. Noveon], beispielsweise Carbopole der Typen 980, 981, 1382, 2984, 5984, ETD 2001, ETD 2020, ETD 2050, Ultrez 10, jeweils einzeln oder in Kombination.
Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.
Vorteilhafte Moisturizer im Sinne der vorliegenden Erfindung sind beispielsweise Glycerin, Panthenol, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure, Aminosäuren und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zur Prophylaxe und Behandlung kosmetischer oder dermatologischer Hautveränderungen, wie sie z. B. bei der Hautalterung auftreten, verwendet werden.
Die erfindungsgemäßen kosmetischen und/oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Nylonpartikel, Pigmente, die weder hauptsächlich UV-Filter- noch färbende Wirkung haben (wie z. B. Bornitrid etc.) und/oder Aerosile^{®} (CAS-Nr. 7631-86-9).

Es ist ebenfalls vorteilhaft, den Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.
Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Retinoide wie zum Beispiel Retinol, Retinal und/oder Retinoësäure und die jeweiligen Ester, α-Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, -2-Aminopropionsäuredi-essigsäure, Flavonoide, Polyphenole, Catechine, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoëharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 0,1 -10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.
Dementsprechend enthalten die Zubereitungen im Sinne der vorliegenden Erfindung vorzugsweise mindestens eine weitere UV-A-, UV-B- und/oder Breitbandfiltersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.
Die erfindungsgemäßen Zubereitungen können ferner vorteilhaft auch in Form von sogenannten ölfreien kosmetischen oder dermatologischen Emulsionen vorliegen, welche eine Wasserphase und mindestens eine bei Raumtemperatur flüssige UV-Filtersubstanz als weitere Phase enthalten.
Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Octyl Salicylate) und Ester der Zimtsäure,vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate).

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).
Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.
Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.
Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.
Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.
Erfindungsgemäß geeignete Zinkoxidpartikel und Vordispersionen von Zinkoxidpartikeln sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |

Geeignete Titandioxidpartikel und Vordispersionen von Titandioxidpartikeln sind unter folgenden Handelsbezeichnungen bei den aufgeführten Firmen erhältlich:

| **Handelsname** | **Coating** | **Hersteller** |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 | Octyltrimethylsilan | Degussa |
| (Uvinul TiO₂) | | |

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.
Vorteilhafte weitere UV-Filtersubstanzen im Sinne der vorliegenden Erfindung sind sulfonierte, wasserlösliche UV-Filter, wie z. B.:
• Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5.'-tetrasulfonsäure-bis-natriumsalz mit der INCI-Bezeichnung Bisimidazylate (CAS-Nr.: 180898-37-7), welches beispielsweise unter der Handelsbezeichnung Neo Heliopan AP bei Haarmann & Reimer erhältlich ist;
• Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
• 1,4-di(2-oxo-10-Sulfo-3-bornylidenmethyl)-Benzol (auch: 3,3'(1,4.Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
• Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.
Vorteilhafte Breitbandfilter oder UV-B-Filtersubstanzen sind beispielsweise Triazinderivate, wie z. B.
• 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist;
• Diethylhexylbutylamidotriazon (INCI: Diethylhexylbutamidotriazone), welches unter der Handelsbezeichnung UVASORB HEB bei Sigma 3V erhältlich ist;
• 4,4',4"-(1,3,5-Triazin-2,4,6-triyltriimino)-tris-benzoësäure-tris(2-ethylhexylester); auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird.

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.
Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches unter der Handelsbezeichnung Mexoryl® XL bei der Fa. Chimex erhältlich ist.

Die weiteren UV-Filtersubstanzen können öllöslich oder wasserlöslich sein.
Vorteilhafte öllösliche UV-B- und/oder Breitband-Filtersubstanzen im Sinne der vorliegenden Erfindung sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.
■ 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX bei Hoffmann La Roche erhältlich ist.

Vorteilhafte wasserlösliche Filtersubstanzen sind z. B.:
Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 erhältlich ist.
Besonders vorteilhafte Zubereitungen im Sinne der vorliegenden Erfindung, die sich durch einen hohen bzw. sehr hohen UV-A- und/oder UV-B-Schutz auszeichnen, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethylhexyloxy)-2-hydroxy]-phenyl}-6-(4-methoxyphenyl)-1,3,5-triazin, jeweils einzeln oder in beliebigen Kombinationen miteinander.
Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Erfindungsgemäß können die erfindungsgemäßen kosmetischen und/oder dermatologischen Zubereitungen wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.
Entsprechend können kosmetische und/oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Handcréme, Nährcrème, Tages- oder Nachtcrème, Reinigungsgele, Gessichtswässer, Stiftzubereitungen sowie als Haarshampoo, Haarkur Körperreinigungsprodukt usw. Es ist gegebenenfalls möglich und vorteilhaft, die erfindungsgemäßen Zusammensetzungen als Grundlage für pharmazeutische Formulierungen zu verwenden.

Zur Anwendung werden die kosmetischen und dermatologischen Zubereitungen erfindungsgemäß in der für Kosmetika üblichen Weise auf die Haut und/oder die Haare in ausreichender Menge aufgebracht.
Auch ist die Verwendung der erfindungsgemäßen kosmetischen und/oder dermatologischen Zubereitungen zur Prophylaxe und Behandlung der Symptome von Altershaut, zur Verhinderung und Verminderung der Entstehung und Ausbeitung von Fältchen und Falten sowie zur Behandlung und Pflege gealterter Haut erfindungsgemäß.
Nicht zuletzt ist die Verwendung der erfindungsgemäßen kosmetischen und/oder dermatologischen Zubereitungen zur Prophylaxe, Behandlung und Reinigung fettiger Haut sowie zur Prophylaxe und Behandlung von unreiner Haut und Akne erfindungsgemäß.
Es ist bei all diesem im Einzelfalle möglich, dass die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so dass er weiß, dass bei solchen Über- oder Unterschreitungen der Boden der vorliegenden Erfindung nicht verlassen wird.

Nachfolgend werden die vorteilhaften Verfahren zur Einarbeitung der Wirkstoffe in die Wachspartikel beschrieben:
1. Zugabe des Polyols zu einer Schmelze des Matrixmaterials (Wachs). Anschließend Zugabe des Wirkstoffes. Die Temperatur der Gesamtschmelze muß so gewählt werden, dass der Wirkstoff vorzugsweise gelöst oder zumindest fein suspendiert ist.
2. Lösen des Wirkstoffes in einem geeigneten Solubilisator, z.B. Polyol. Diese Mischung wird der Schmelze des Matrixmaterials zugefügt und erhitzt, bis das gesamte System homogen ist.

Das Herstellungsprocedere der wirkstoffhaltigen Wachspartikel lässt beispielhaft sich wie folgt beschreiben.
Das Polyol wird zusammen mit dem Wachs eingewogen und aufgeschmolzen. Die Wasserphase wird, ggf. unter Zusatz von Additiven (z.B. Konservierungsmittel), separat auf ca. 80°C erhitzt. Zugabe des Wirkstoffes zur Wachs/Polyolschmelze (Verfahren 1) und erhitzen unter Rühren bis das System homogen ist. Dann wird die geschmolzene Wachsphase unter intensivem Rühren zur heißen Wasserphase gegeben. Wenn eine oberflächenaktive Substanz (Emulgator) zur Verbesserung der Dispergierbarkeit der Wachspartikel eingesetzt werden soll, kann er vorher zur Wachs- oder zur Wasserphase gegeben werden. Welche Vorgehensweise geeigneter ist, muß für jedes System individuell geprüft werden. Die Dispergierung kann mit Hilfe von bekannten Verfahren (Ultraturrax, Becomix, Blattrührer, Ultraschall) durchgeführt werden. Die hierdurch erhaltene wässrige Dispersion liefert nach Abkühlen auf Raumtemperatur üblicherweise Wachspartikel im Mikrometerbereich (1 - 250 µm). Bei Bedarf kann sich dieser Dispergierung eine Hochdruckhomogenisierung anschließen, wodurch Teilchen im Nanometerbereich entstehen (10 nm - ca. 5µm). Je nach Wirkstoff muß anschließend noch der pH-Wert der Wasserphase durch Zugabe von Säuren oder Basen auf den gewünschten Wert eingestellt werden.

Die nach diesem Verfahren hergestellten Wachsdispersionen zeigen eine gute LangzeitStabilität der Dispersion, z.B. bei Lagerung über einen Zeitraum von einem Monat bei Raumtemperatur.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bzw. Trägersysteme bezogen.

### Beispiel 1:

Herstellung einer Suspension von Acetylsalicylsäure-haltigen Mikropartikeln

| | |
|---|---|
| 10g | Kesterwachs K48P (C18-C22 Alkylhydroxystearoylstearat) |
| 2,5g | Eucerit (Wollwachs) |
| 0,25g | Acetylsalicylsäure |
| 0,25g | Dipropylenglycol |
| 3g | Triceteareth-4-phosphat |
| 0,5g | Konservierungsmittel (Phenoxyethanol und Parabene) |
| 83,5g | Wasser, deminaralisiert |

Das Kesterwachs K48P, das Eucerit und das Triceteareth-4-phosphat werden bei ca. 60 °C geschmolzen. Anschließend wird das Dipropylenglycol und danach die Acetylsalicylsäure zugesetzt und dann unter langsamer Temperaturerhöhung solange gerührt, bis der gesamte Wirkstoff gelöst ist. Unter kontinuierlichem Rühren mit einem Blattrührer wird die Lipidphase in die auf 80°C erhitzten Wasserphase, welche das das Konservierungsmittel enthält, gegeben und somit eine Voremulsion hergestellt. Diese Voremulsion wird mittels Ultraturrax (ca. 2 Minuten bei 10000 UpM) intensiv dispergiert und auf Raumtemperatur abgekühlt, wobei man eine milchige Suspension erhält.

### Beispiel 2:

Herstellung einer Suspension von Mikropartikeln mit Grünem Tee Konzentrat

| | |
|---|---|
| 10g | Kesterwachs K48P (C18-C22 Alkylhydroxystearoylstearat) |
| 3g | Eucerit (wollwachs) |
| 0,2g | Grüner Tee Konzentrat, pulverförmig (Camellia sinensis) |
| 0,3g | Dipropylenglycol |
| 5g | Polyglyceryl-3 Methylglucosedistearat |
| 0,5g | Konservierungsmittel (Phenoxyethanol und Parabene) |
| 81g | Wasser, demineralisiert |

Das Kesterwachs K48P, das Eucerit und der Emulgator Polyglyceryl-3 Methylglucosedistearat werden bei ca. 70 °C geschmolzen. Anschließend wird die Mischung aus Dipropylenglycol und Grünem Tee Pulver zugesetzt und dann unter langsamer Temperaturerhöhung solange gerührt, bis der gesamte Wirkstoff gelöst ist. Unter kontinuierlichem Rühren mit einem Blattrührer wird die Lipidphase in die auf 80°C erhitzten Wasserphase, welche das das Konservierungsmittel enthält, gegeben und somit eine Voremulsion hergestellt. Diese Voremulsion wird mittels Ultraturrax (ca. 2 Minuten bei 10000 UpM) intensiv dispergiert und auf Raumtemperatur abgekühlt, wobei man eine milchige Suspension erhält.
Hochdruckhomogenisierung bei 150 bar (gerätetyp Ariete NS 2006 L, Firma Niro Soavi) liefert die feindisperse Suspension von wirkstoffhaltigen Wachspartikeln.

### Beispiel 3:

Herstellung einer Suspension von Mikropartikeln mit Grünem Tee Konzentrat

| | |
|---|---|
| 7,5g | Candelilla Wachs |
| 2,5g | Eucerit (Wollwachs) |
| 0.1g | Liponamid |
| 0,5g | Dipropylenglycol |
| 3g | Polyglyceryl-3 Methylglucosedistearat |
| 0,5g | Konservierungsmittel (Phenoxyethanol und Parabene) |
| 85,9g | Wasser, deminaralisiert |

Das Candilillawachs, das Eucerit und der Emulgator Polyglyceryl-3 Methylglucosedistearat werden bei ca. 90 °C geschmolzen. Anschließend wird die Mischung aus Dipropylenglycol und Liponamid zugesetzt und dann unter langsamer Temperaturerhöhung solange gerührt, bis der gesamte Wirkstoff gelöst ist. Unter kontinuierlichem Rühren mit einem Blattrührer wird die Lipidphase in die auf 80°C erhitzten Wasserphase, welche das das Konservierungsmittel enthält, gegeben und somit eine Voremulsion hergestellt. Diese Voremulsion wird mittels Ultraturrax (ca. 2 Minuten bei 10000 UpM) intensiv dispergiert und auf Raumtemperatur abgekühlt, wobei man eine milchige Suspension erhält.
Hochdruckhomogenisierung bei 150 bar (gerätetyp Ariete NS 2006 L, Firma Niro Soavi) liefert die feindisperse Suspension von wirkstoffhaltigen Wachspartikeln.

### Beispiele 4-13: O/W-Cremes

| **Beispiel Nummer** | **4** | **5** | **6** | **7** | **8** |
|---|---|---|---|---|---|
| **(Gew.%)** | | | | | |
| Glycerylstearatcitrat | 2 | | | | |
| Glycerylsterat, selbstemulgierend | | 5 | 3 | 1 | 2 |
| PEG-40-Stearat | | | 1 | | |
| Stearinsäure | | | | 4 | |
| Myristylmyristat | 1 | | | | 1 |
| Behenylalkohol | | | | 1 | |
| Stearylalkohol | 2 | 1 | | | |
| Cetearylalkohol | | | | | 2 |
| Cetylalkohol | 1 | | 3 | 3 | |
| Hydrierte Kokosfettglyceride (Hydrogenated Coco Glycerides) | 2 | | | | |
| Sheabutter | | 1 | | | 2 |
| C12-15 Alkylbenzoat | | 3 | 2 | | 3 |
| Butylenglycol Dicaprylat/Dicaprat | 1 | | | 1 | |
| Caprylsäure/Caprinsäure Triglycerid | | 1 | 2 | | 2 |
| Ethylhexylkokosfettsäureester | 3 | | | | 1 |
| Octyldodecanol | | | 1 | | |
| Jojobaöl | | | | 1 | |
| Mineralöl | | 1 | | | |
| Vaseline | 1 | | 1 | | 2 |
| Cyclohexasiloxan | 3 | | 4 | | 5 |
| Dimethicon | | 3 | 1 | | |
| Dicaprylylether | 1 | 3 | 2 | | |
| Dicarprylylcarbonat | | | | 3 | |
| TiO₂ | | | 1 | | 1 |
| Ethylhexylmethoxycinnamat | 5 | 3 | 5 | | 3 |
| Ethylhexyltriazon | | 1 | | | |
| Ethylhexylcyanodiphenyl-acrylat (Octocrylen) | | | | 5 | 3 |
| Butylrriethoxydibenzoylmethan | 1 | | | | |
| Bis-Ethylhexyloxyphenol-methoxyphenyltriazine | | 1 | | | |
| Ethylhexylsalicylat | 1 | | | | |
| 2-Hydroxy-4-Methoxy- benzophenon (Oxybenzone) | | | 2 | 3 | 2 |
| Phenylbenzimidazol sulfonsäure | | | | 2 | |
| Acetylsalicylsäure (gem. Beispiel 1) | 0,05 | | | | |
| Grüner Tee Extrakt (Beispiel 2) | | 0,1 | | 0,2 | |
| Liponamid (Beispiel 3) | | | 0,05 | | 0,03 |
| Milchsäure | | | 0,3 | | |
| Tocopherylacetat | | | 1 | | 0,3 |
| Citronensäure, Natriumsalz | | 0.1 | | | |
| Ascorbylglucosid | | | | | 0,1 |
| Trinatrium EDTA | | 0.1 | | 0,2 | |
| Iminodisuccinat, Natriumsalz | 0,2 | | 0,1 | | 0,1 |
| Phenoxyethanol | 0,3 | | 0,3 | 0,2 | 0,2 0,3 |
| p-Hydroxybenzoesäurealkylester (Paraben) | 0,6 | 0,3 | 0,2 | 0,3 | 0.3 |
| Hexamidindiisethionat | | 0,04 | | | |
| Diazolidinylharnstoff | 0,25 | | 0,1 | 0,2 | 0,1 |
| 1,3-Dimethylol-5,5-dimethyl-hydahtoin(DMDM Hydantoin) | | 0,2 | | | |
| lodopropynylbutylcarbamat | | 0,1 | | | |
| Ethanol denaturiert | | 2 | | | |
| Xanthan Gummi | | | 0,1 | | |
| Polyacrylsäure (Carbomer) | 0,05 | | | | 0,1 |
| Ammoniumpolyacryloyldimethyltaurate | 0,4 | | | 0,3 | |
| Ammoniumacryloyldimethyltaurate/ Vinylpyrrolidoncopolymere | | 0,5 | 0,3 | | |
| Aluminium Stärke Octenylsüccinate | | | | | 0,5 |
| Glycerin | 10 | 6 | 7 | 7 | 5 |
| Butylenglycol | | 1 | | 1 | |
| wasser- und/oder öllösliche Farbstoffe | 0,05 | | | | |
| Füllstoffe/ Additive (Distärke-phosphat, SiO₂, BHT, Talkum, Aluminiumstearat) | 0,1 | 1 | 0,2 | 0,5 | 0,05 |
| Parfüm | q.s. | q.s. | q.s. | q.s. | q.s. |
| Wasser | Ad | Ad | Ad | Ad | Ad |
| | 100 | 100 | 100 | 100 | 100 |

| **Beispiel Nummer** | **9** | **10** | **11** | **12** | **13** |
|---|---|---|---|---|---|
| **(Gew.%)** | | | | | |
| Glycerylsterat, selbstemulgierend | 2,5 | | | | 1 |
| PEG-40-Stearat | 1 | | | | |
| Polyglyceryl-3-Methylglucosedistearat | | 3 | | | |
| Sorbitanstearat | | 1 | | | |
| Polyethylenglycol(21)stearyl-ether (Steareth-21) | | | 2 | | |
| Polyethylenglycol(2)stearyl-ether (Steareth-2) | | | 1 | | |
| Cetearylglucosid | | | | 2 | |
| Stearinsäure | | | | | 2,5 |
| Myristylmyristat | | | | 1 | |
| Behenylalkohol | | 2 | | | 2 |
| Stearylalkohol | | | | 4 | |
| Cetearylalkohol | 2 | | 2 | | |
| Cetylalkohol | | 1 | | | 3 |
| Hydrierte Kokosfettglyceride (Hydrogenated Coco Glycerides) | 1 | | | | 1 |
| Sheabutter | 2 | | 1 | | |
| C12-15 Alkylbenzoat | 4 | 2 | 5 | 2 | 2 |
| Butylenglycol Dicaprylat/Dicaprat | | | | | |
| Caprylsäure/Caprinsäure Triglycerid | 1 | 1 | | 3 | |
| Hydriertes Polydecen | | 1 | | 1 | |
| Ethylhexylkokosfettsäureester | | | | | 2 |
| Octyldodecanol | 1 | | 1 | | 2 |
| Mineralöl | | | 1 | | |
| Vaseline | 1 | | | | |
| Cyclohexasiloxan | | 3 | 2 | | 2 |
| Dimethylpolysiloxan (Dimethicon) | | | | | 1 |
| Dicaprylylether | | | 2 | | |
| Dicarprylylcarbonat | | 2 | | 3 | 4 |
| Polydecen | | | | 4 | |
| TiO₂ | 1 | | 1 | | 1 |
| Ethylhexylmethoxycinnamat | | 5 | 4 | 3 | 7 |
| Ethylhexylcyanodiphenyl-acrylat (Octocrylen) | 5 | | | 3 | |
| Ethylhexyltriazon | | | 1 | | |
| Phenylbenzimidazol-sulfonsäure | 0,5 | | | | 1 |
| Butylmethoxydibenzoylmethan | | 2 | | 1 | |
| 2-Hydroxy-4-Methoxy- benzophenon (Oxybenzone) | | | | | 2 |
| Acetylsalicylsäure (gem. Beispiel 1) | 0,1 | | | | 0,05 |
| Grüner Tee Extrakt (Beispiel 2) | | 0,05 | | | |
| Liponamid (Beispiel 3) | | | 0,02 | 0,03 | |
| Tocopherylacetat | | 0,3 | | 1 | 0,5 |
| Biotin | | | 0,02 | | |
| Ascorbinsäure | 0,1 | | | | |
| Lactoferrin | | | | | 0,05 |
| Trinatrium EDTA | | | 0,2 | 0,1 | |
| Iminodisuccinat | 0,2 | 0,2 | | | 0,1 |
| Phenoxyethanol | 0,5 | 0,4 | 0,5 | | 0,3 |
| p-Hydroxybenzoesäurealkylester (Paraben) | 0,1 | | | 0,4 | 0,6 |
| Hexamidindiisethionat | | | 0,1 | | |
| Diazolidinylharnstoff | 0,2 | 0,2 | | 0,1 | |
| Iodopropynylbutylcarbamat | | | 0,25 | | |
| Ethanol denaturiert | | 5 | | | 3 |
| 2-Ethylhexylglycerinether (Octoxyglycerin) | | | | 0,4 | |
| Xanthan Gummi | 0,1 | 0,1 | | 0,1 | |
| Polyacrylsäure (Carbomer) | | | 0,1 | | 0,3 |
| Ammoniumpolyacryloyldimethyltaurate | | | | 0,3 | |
| Ammoniumacryloyldimethyltaurate/Vinylpyrrolidoncopolymere | | 0,5 | 0,3 | | |
| Aluminium Stärke Octenylsuccinate | | | | 0,5 | |
| Glycerin | 8 | 6 | 5 | 7 | 6 |
| Butylenglycol | | | 2 | | |
| wasser- und/oder öllösliche Farbstoffe | | | | | 0,1 |
| Additive (Distärkephosphat, SiO₂, Talkum, BHT, Aluminiumstearat, □-Tocopherol) | 0.03 | 0,1 | 0,05 | 3 | 1 |
| Parfüm | q.s. | q.s. | q.s. | q.s. | q.s. |
| Wasser | Ad 100 | Ad 100 | Ad 100 | Ad 100 | Ad 100 |

### Beispiel 14: Hydrodispersion/Gelcreme

| | **(Gew.%)** |
|---|---|
| Cetylalkohol | 2 |
| Sheabutter | 2 |
| Caprylsäure/Caprinsäure Triglycerid | 2 |
| Octyldodecanol | 2 |
| Cyclohexasiloxan | 4 |
| Dimethylpolysiloxan (Dimethicon) | 1 |
| Polydecen | 2 |
| Ethylhexylmethoxycinnamat | 3 |
| Bis-Ethylhexyloxyphenol-methoxyphenyltriazin | 0,5 |
| Grüner Tee Extrakt (Beispiel 2) | 0,2 |
| Natriumascorbylphosphat | 0,05 |
| Iminodisuccinat | 0,2 |
| Phenoxyethanol | 0,4 |
| p-Hydroxybenzoesäurealkyleste (Paraben) | 0,4 |
| Vernetztes Alkylacrylat (Alkylacrylate Cosspolymer) | 0,2 |
| Glycerin | 6 |
| Parfüm | q.s. |
| Wasser | Ad 100 |

### Beispielrezepturen 15 - 18; Foundations

| **Inhaltsstoffe (Gew.%)** | **15** | **16** | **17** | **18** |
|---|---|---|---|---|
| PEG-30-stearat | 1,5 | | | |
| Gylcerylstearat | 0,5 | | | 2 |
| Ceteareth-20 | 1 | | | |
| Polyglyceryl-3 Methylglucose stearat | | | 3,5 | |
| Sorbinsäurestearat | | | 1,5 | |
| Stearinsäure | 2 | | | 1,8 |
| Glycerylstearatcitrat | | 3,5 | | |
| Cetylalkohol | 0,5 | 0,75 | 1,0 | |
| Lecithin | | | | 0,5 |
| Veegum K = Mg-Al-Silicate | 0,8 | | | 1 |
| Xanthan Gum | | | | 0,2 |
| Carbomer | | 0,2 | | |
| Hydroxyethylcellulose | | | 0,1 | |
| Ammoniumacryloyldimethyltaurate/Vinylpyrrolidoncopolymere | | 0,4 | | |
| Aluminium Stärke Octenylsuccinate | | | | 1 |
| Caprylsäure / Caprinsäure Triglycerid | | 2 | 3 | 2 |
| Dicaprylylether | | 2 | 3 | 3 |
| Octyldodecanol | | | 3 | |
| Dimethicon | 3 | | 3 | 3 |
| Cyclomethicon | | 4 | 3 | 2 |
| C12-C15 Alkyl Benzoate | 2 | | | |
| Cetearyloctanoat | 2 | | | |
| Squalan | 1 | | | 6 |
| Isopropylpalmitat | 1 | | | 2 |
| PPG -15 Stearylether | 2 | 3 | | |
| Hydrierete Kokos-Glyceride | 2 | | | |
| Stearyldimethicon | 9 | | | |
| Acetyliertes Lanolinöl | 2 | 0,2 | | |
| Ethylhexylmethoxycinnamat | 2 | 2 | | 2 |
| TiO₂ | | 2 | | 2 |
| Ethylhexyltriazon | 2 | | | 1 |
| Bis-Ethylhexyloxyphenol-methoxyphenyltriazin | | 1 | 2 | |
| Ubichinon (Q10) | | | 0,02 | |
| Tocopherylacetat | | | | 0,5 |
| Biotin | 0,01 | | | |
| Grüner Tee Extrakt (Beispiel 2) | 0,02 | 0,05 | 0,1 | 0,03 |
| Silikon | | 0.8 | | |
| Nylon-12 | | | 5 | |
| Lauroyllysin | 0,5 | | 0,5 | |
| Kaolin | 0,5 | | 1 | 2 |
| Eisenoxide | 1,2 | 2,4 | 2,6 | 3 |
| Titandioxid | 3,8 | 5,6 | 4,5 | 6,5 |
| Interferenz Pigmente | 0,2 | | | 0,5 |
| Pigment Low Lustre | 0,2 | | | 0,2 |
| ZnO | | 1 | | |
| Polymethylsilsesquioxan (Tospearl 2000B) | | 2 | | |
| EDTA | 0,1 | 0,6 | 1 | 1 |
| Glycerin | 2 | 5 | 5 | 10 |
| Phenoxyethanol und Paraben (Phenonip) | 1 | 0,5 | | 1 |
| Imidazonidinylharnstoff | 0,3 | 0,3 | | 0,25 |
| Neutralisationsmittel | q.s. | q.s. | q.s. | q.s. |
| Parfum, Antioxidantien | q.s. | q.s. | q.s. | q.s. |
| Wasser | ad 100 | ad 100 | ad 100 | ad 100 |

### Beispiele 19 - 22; Pickering

| **(Gew.%)** | **19** | **20** | **21** | **22** |
|---|---|---|---|---|
| Mikrokristallines Wachs | 4,5 | 4 | 2,6 | 1,5 |
| Ozokerit | | 0,5 | 0,5 | 0,5 |
| Carnaubawachs | 1,5 | 2,0 | 1,5 | 1,8 |
| Candelillawachs | 4,0 | 2,0 | 2,0 | 6,0 |
| Bienenwachs | | 1,0 | | |
| C24-40 Alkyl Stearat | | | 2,0 | 1,5 |
| Lauryl Pyrrolidoncarbonsäure | | 3,0 | | |
| Lanolinöl | 4,0 | | | 2,0 |
| Bis-Diglyceryl Polyacyladipat-2 | 3,5 | 5,0 | | |
| Simethicon | 1,0 | | 0,5 | |
| Isopropyl Palmitat | 3,5 | 4,0 | | 2,0 |
| Triisostearin | 3,0 | | | |
| Myristyl Lactat | 4,0 | | 2,0 | 4,0 |
| Jojobaöl | 1,0 | | | 3,0 |
| Diisostearyl Malat | | | | 1,2 |
| Caprylic/Capric Triglycerid | | 4,0 | 2,0 | |
| Pentaerythrityl Tetraisostearat | | | 2,0 | |
| Isodecyl Neopentanoat | | 2,0 | | |
| Dicaprylyl Carbonat | | 2,0 | 2,0 | 3,0 |
| C12-15 Alkyl Benzoat | | | | 8,0 |
| Hydrogeniertes Polydecen | 2,5 | | | |
| Squalan | | | 2,0 | |
| Octyldodecanol | 2,5 | | ad 100 | 3,0 |
| Diisostearyl Fumarat | | | | ad 100 |
| PVP/Hexadecen Copolymer | | 0,5 | | |
| Acetylsalicylsäure (Beispiel 1) | 0,03 | | | |
| Grüner Tee Extrakt (Beispiel 2) | | 0,05 | | 0,1 |
| Liponamid (Beispiel 3) | | | 0,02 | |
| Ethylhexyl Methoxycinnamat | 2,0 | 1,0 | | 2,0 |
| Butyl Methoxydibenzoylmethan | | 0,2 | | |
| Bis-Ethylhexyloxyphenol Methoxyphenyl Triazin | | | | 0,5 |
| Mikronisiertes Titandioxid (Eusolex T 2000) | 2,0 | 2,0 | 3,0 | |
| Silica Dimethyl Silylat (Aerosil R972) | | 0,3 | | |
| Polymethylsilsesquioxan (Tospearl 2000B) | | | 1,0 | 3,0 |
| Interferenz Pigmente | | 4,0 | 3,0 | |
| Titandioxid CI 77891 | 4,0 | 3,8 | 2,0 | 3,0 |
| Eisenoxide CI 77491, 77492, 77499 | 3,2 | 2,2 | | 2,2 |
| D&C Rot 7 | 0,6 | | 1,9 | 1,3 |
| D&C Rot 6 | | | 0,4 | |
| FD&C Blau 1 | | | | 0,3 |
| D&C Rot 33 | | 0,3 | 2,2 | |
| D&C Rot 21 | | | | 0,5 |
| FD&C Gelb 6 | | 1,5 | | |
| D&C Rot 34 | | | 0,3 | 0,8 |
| Tocopheryl Acetat | 1,0 | | 1,5 | |
| Ubichinon | | | 0,1 | |
| Xylitol | 2,0 | | 2,0 | 2,0 |
| Glycerin | 4,0 | 3,0 | 6,0 | 5,0 |
| Konservierungsmittel, BHT, Parfum, Aroma | q.s. | q.s. | q.s. | q.s. |
| Wasser | 30,0 | 40,0 | 50,0 | 20,0 |
| Ricinusöl | ad 100 | ad 100 | 5,0 | |

## Patentansprüche

1. Trägersystem für kosmetische oder pharmazeutische Wirkstoffe, umfassend Wachsmikro- oder nanopartikel aus einem oder mehreren Wachsen oder Wachs/Ölgemischen enthaltend 0,01 bis 15 Gew.%, bezogen auf die Wachsphase, einen oder mehrere Solubilisatoren gewählt aus der Gruppe der (a) Polyole, (b) Polyethylenglycole, und/oder (c) Wollwachsalkohole und einen oder mehrere kosmetische und/oder pharmazeutische Wirkstoffe.

2. Trägersystem nach Anspruch 1, **dadurch gekennzeichnet, dass** als Polyole (a) Butylenglycol, Dipropylenglycol, Propylenglycol und/oder Polyolprepolymer gewählt werden.

3. Trägersystem nach Anspruch 1 oder 2, **dadurch gekennzeichnet, dass** die Polyethylenglycole (b) einen mittleren Ethylenglycolanteil von 8-500 Einheiten besitzen.

4. Trägersystem nach Anspruch 1, 2 oder 3, **dadurch gekennzeichnet, dass** als Wollwachsalkohol (c) Eucerit gewählt wird.

5. Trägerssystem nach einem der vorhergehenden Ansprüche, wobei der oder die Wirkstoffe hydrophile Substanzen mit einem log P_{*Octanol*/}*_{Wasser}* kleiner 3 sind.

6. Trägersystem nach einem der vorstehenden Ansprüche, **dadurch gekennzeichnet, dass** die Wirkstoffe gewählt werden aus der Gruppe Carnitin und deren Derivate, Creatin und deren Derivate, Liponamid, Salicylsäure und deren Derivate, Acetylsalicylsäure, Isoflavonoide, insbesondere Genistein und Daidzein, Polyphenole des Grünen Tees, insbesondere Epigallocatechingallat , Rutin und seine Derivate, Ester und/oder Amide von Aminosäuren, Peptide und deren Derivate bestehend aus 2-10 Aminosäuren, Ascorbinsäure und/oder deren Derivate, Vitamine der B-Gruppe, Niacinamid, Pyridoxin und/oder Folsäure.

7. Trägersystem nach einem der vorstehenden Ansprüche, **dadurch gekennzeichnet, dass** die Wirkstoffe gewählt werden aus der Gruppe Analgetika, Antirheumatika, z.B. Ibuprofen, Diclofenac, außerdem Peptidhormone, Vasodilatoren, Antiallergica, z.B. Terfenadin, weiterhin Antibiotika, Chemotherapeutika, Makrolide, Aminoglycoside, Cefalosporine, Penicilline, Polypeptide, Tetracycline, Virustatika Antimykotika, Opthalmika, Psychopharmaka, Immunstoffe und/oder Cytostatika.

8. Trägersystem nach einem der vorstehenden Ansprüche, **dadurch gekennzeichnet, dass** die Wachse gewählt werden aus der Gruppe Cetylpalmitat, Candellilawachs, Carnaubawachs, Paraffinwachs, Tripalmitin (Dynasan 116), C15-C40 Alkyl Stearylstearate, Bienenwachs und/oder Beerenwachs.

9. Trägersystem nach einem der vorstehenden Ansprüche, **dadurch gekennzeichnet, dass** die Wachspartikel eine Partikelgröße von 0,01 bis 250 µm aufweist.

10. Trägersystem nach Anspruch 9, **dadurch gekennzeichnet, dass** die Wachspartikel einen mittleren Partikelgröße von 0,3 bis 30 µm aufweist.

11. Kosmetische und/oder dermatologische wirkstoffhaltige Zubereitungen enthaltend ein Trägersystem nach einem der vorstehenden Ansprüche.

12. Zubereitung nach Anspruch 11 enthaltend eine oder mehrere oberflächenaktive Verbindungen.

13. Zubereitung nach Anspruch 11 oder 12 enthaltend Moisturizer, Antioxidantien und/oder UV-Schutzsubstanzen.

14. Verwendung des Trägersystems nach Anspruch 1 bis 10 zur Verkapselung von hydrophilen kosmetischen oder pharmazeutischen Wirkstoffen in kosmetischen und/oder dermatologischen Zubereitungen.

15. Verwendung des Trägersystems nach Anspruch 14 , **dadurch gekennzeichnet, dass** die Wirkstoffe einen log P_{*Octanol*/*Wasser*} kleiner 3 aufweisen.

## Claims

1. Carrier system for cosmetic or pharmaceutical active ingredients, comprising wax microparticles or nanoparticles from one or more waxes or wax/oil mixtures comprising 0.01 to 15% by weight, based on the wax phase, of one or more solubilizers selected from the group of (a) polyols, (b) polyethylene glycols and/or (c) wool wax alcohols and one or more cosmetic and/or pharmaceutical active ingredients.

2. Carrier system according to Claim 1, **characterized in that** the polyols (a) selected are butylene glycol, dipropylene glycol, propylene glycol and/or polyol prepolymer.

3. Carrier system according to Claim 1 or 2, **characterized in that** the polyethylene glycols (b) have an average ethylene glycol fraction of 8-500 units.

4. Carrier system according to Claim 1, 2 or 3, **characterized in that** the wool wax alcohol (c) selected is eucerit.

5. Carrier system according to one of the preceding claims, where the active ingredient or ingredients are hydrophilic substances with a log P_{octanol/water} of less than 3.

6. Carrier system according to one of the preceding claims, **characterized in that** the active ingredients are selected from the group consisting of carnitine and derivatives thereof, creatine and derivatives thereof, liponamide, salicylic acid and derivatives thereof, acetylsalicylic acid, isoflavonoids, in particular genistein and daidzein, polyphenols of green tea, in particular epigallocatechin gallate, rutin and its derivatives, esters and/or amides of amino acids, peptides and derivatives thereof, consisting of 2 - 10 amino acids, ascorbic acid and/or derivatives thereof, vitamins of the B group, niacinamide, pyridoxine and/or folic acid.

7. Carrier system according to one of the preceding claims, **characterized in that** the active ingredients are selected from the group consisting of analgesics, antirheumatics, e.g. ibuprofen, diclofenac, moreover peptide hormones, vasodilators, antiallergics, e.g. terfenadine, also antibiotics, chemotherapeutics, macrolides, aminoglycosides, cefalosporins, penicillins, polypeptides, tetracyclines, virustatics, antimycotics, opthalmics, psychopharmaceuticals, immune substances and/or cytostatics.

8. Carrier system according to one of the preceding claims, **characterized in that** the waxes are selected from the group consisting of cetyl palmitate, candellila wax, carnauba wax, paraffin wax, tripalmitin (Dynasan 116), C15 - C40 alkyl stearylstearates, beeswax and/or berry wax.

9. Carrier system according to one of the preceding claims, **characterized in that** the wax particle has a particle size of from 0.01 to 250 µm.

10. Carrier system according to Claim 9, **characterized in that** the wax particle has an average particle size of from 0.3 to 30 µm.

11. Cosmetic and/or dermatological active-ingredient-containing preparations comprising a carrier system according to one of the preceding claims.

12. Preparation according to Claim 11, comprising one or more surface-active compounds.

13. Preparation according to Claim 11 or 12, comprising moisturizers, antioxidants and/or UV protection substances.

14. Use of the carrier system according to Claims 1 to 10 for encapsulating hydrophilic cosmetic or pharmaceutical active ingredients in cosmetic and/or dermatological preparations.

15. Use of the carrier system according to Claim 14, **characterized in that** the active ingredients have a log P_{octanol/water} of less than 3.

## Revendications

1. Système véhicule pour substances actives cosmétiques ou pharmaceutiques, comprenant des micro- ou nanoparticules de cire à base d'une ou plusieurs cires ou d'un ou plusieurs mélanges cire/huile, contenant de 0,01 à 15 % en poids, par rapport à la phase de cire, d'un ou plusieurs solubilisants choisis dans le groupe des (a) polyols, (b) polyéthylèneglycols, et/ou (c) alcolanums et une ou plusieurs substances actives cosmétiques et/ou pharmaceutiques.

2. Système véhicule selon la revendication 1, **caractérisé en ce qu'**on choisit comme polyols (a) le butylèneglycol, le dipropylèneglycol, le propylèneglycol et/ou un prépolymère de polyol.

3. Système véhicule selon la revendication 1 ou 2, **caractérisé en ce que** les polyéthylèneglycols (b) ont une teneur moyenne en éthylèneglycol de 8-500 unités.

4. Système véhicule selon la revendication 1, 2 ou 3, **caractérisé en ce qu'**on choisit comme alcolanum (c) l'eucérite.

5. Système véhicule selon l'une quelconque des revendications précédentes, dans lequel la ou les substance(s) active(s) est/sont une/des substance(s) hydrophile (s) ayant un log P_{octanol/eau} inférieur à 3.

6. Système véhicule selon l'une quelconque des revendications précédentes, **caractérisé en ce qu'**on choisit les substances actives dans le groupe constitué par la carnitine et ses dérivés, la créatine et ses dérivés, le liponamide, l'acide salicylique et ses dérivés, l'acide acétylsalicylique, des isoflavonoïdes, en particulier la génistéine et la daidzéine, les polyphénols du thé vert, en particulier le gallate d'épigallocatéchol, la rutine et ses dérivés, des esters et/ou amides d'acides aminés, des peptides et leurs dérivés constitués de 2-10 acides aminés, l'acide ascorbique et/ou ses dérivés, des vitamines du groupe B, le niacinamide, la pyridoxine et/ou l'acide folique.

7. Système véhicule selon l'une quelconque des revendications précédentes, **caractérisé en ce que** les substances actives sont choisies dans le groupe constitué par les analgésiques, les antirhumatismaux, par exemple l'ibuprofène, le diclofénac, en outre les hormones peptidiques, les vasodilatateurs, les antiallergiques, par exemple la terfénadine, en outre les antibiotiques, les agents chimiothérapeutiques, les macrolides, les aminoglycosides, les céphalosporines, les pénicillines, des polypeptides, les tétracyclines, les virostatiques, les antimycosiques, les agents ophtalmiques, les agents psychopharmaceutiques, les substances immunitaires et/ou les agents cytostatiques.

8. Système véhicule selon l'une quelconque des revendications précédentes, **caractérisé en ce que** les cires sont choisies dans le groupe constitué par le palmitate de cétyle, la cire de candelilla, la cire de carnauba, la cire de paraffine, la tripalmitine (Dynasan 116), les stérylstéarates d'alkyle en C₁₅-C₄₀, la cire d'abeille et/ou la cire de baies.

9. Système véhicule selon l'une quelconque des revendications précédentes, **caractérisé en ce que** la particule de cire présente une taille de particule de 0,01 à 250 µm.

10. Système véhicule selon la revendication 9, **caractérisé en ce que** la particule de cire présente une taille moyenne de particule de 0,3 à 30 µm.

11. Préparations cosmétiques et/ou dermatologiques contenant des substances actives, qui contiennent un système véhicule selon l'une quelconque des revendications précédentes.

12. Préparation selon la revendication 11, contenant un ou plusieurs composés tensioactifs.

13. Préparation selon la revendication 11 ou 12, contenant des agents hydratants, des antioxydants et/ou des substances protectrices contre les UV.

14. Utilisation du système véhicule selon l'une quelconque des revendications 1 à 10, pour l'encapsulation de substances actives cosmétiques ou pharmaceutiques hydrophiles dans des préparations cosmétiques et/ou dermatologiques.

15. Utilisation du système véhicule selon la revendication 14, **caractérisée en ce que** les substances actives présentent un log P_{octanol/eau} inférieur à 3.
